# EUROPEAN PATENT SPECIFICATION

(11) **EP 4 366 749 B1**
(45) Date of publication and mention of the grant of the patent: **13.08.2025**
(21) Application number: 22755287.4
(22) Date of filing: 05.07.2022
(51) Int. Cl.: A61K 35/741, A61P 11/00

(54) **CORYNEBACTERIUM STRAINS FOR USE IN THE PREVENTION OF A SARS-COV-2 INFECTION**
CORYNEBACTERIUM STÄMME ZUR VERWENDUNG BEI DER PRÄVENTION EINER SARS-COV-2 INFEKTION
SOUCHES DE CORYNEBACTERIUM DESTINÉES À ÊTRE UTILISÉES DANS LA PRÉVENTION D'UNE INFECTION SARS-COV-2

(30) Priority: 09.07.2021 HU 2100261
(43) Date of publication of application: 15.05.2024
(73) Proprietor: Semmelweis Egyetem, 1085 Budapest (HU)
(72) Inventor: SZABÓ, Dóra, Budapest /1022 (HU); TAMÁS, László, Budapest / 1161 (HU); OSTORHÁZI, Eszter, Diósd / 2049 (HU); KRISTÓF, Katalin, Budapest /1085 (HU); MERKELY, Béla, Budapest / 1022 (HU)
(74) Representative: Danubia Patent & Law Office LLC
(86) International application number: PCT/HU2022/050057
(87) International publication number: WO 2023/281284

(56) References cited:
- DATABASE MEDLINE [online] US NATIONAL LIBRARY OF MEDICINE (NLM), BETHESDA, MD, US; 20 November 2020 (2020-11-20), MOSTAFA HEBA H ET AL: "Metagenomic Next-Generation Sequencing of Nasopharyngeal Specimens Collected from Confirmed and Suspect COVID-19 Patients.", Database accession no. NLM33219095
- MOSTAFA HEBA H ET AL: "Metagenomic Next-Generation Sequencing of Nasopharyngeal Specimens Collected from Confirmed and Suspect COVID-19 Patients.", MBIO 20 11 2020, vol. 11, no. 6, 20 November 2020 (2020-11-20), ISSN: 2150-7511
- PHILLIPA ENGEN ET AL: "Nasopharyngeal Microbiota in SARS-CoV-2 Positive and Negative Patients", BIOLOGICAL PROCEDURES ONLINE, BIOMED CENTRAL LTD, LONDON, UK, vol. 23, no. 1, 1 June 2021 (2021-06-01), pages 1 - 6, XP021291580, DOI: 10.1186/S12575-021-00148-6
- ROSAS-SALAZAR CHRISTIAN ET AL: "SARS-CoV-2 infection and viral load are associated with the upper respiratory tract microbiome", JOURNAL OF ALLERGY AND CLINICAL IMMUNOLOGY, ELSEVIER, AMSTERDAM, NL, vol. 147, no. 4, 9 February 2021 (2021-02-09), pages 1226, XP086528992, ISSN: 0091-6749, [retrieved on 20210209], DOI: 10.1016/J.JACI.2021.02.001
- MOSTAFA HEBA H ET AL: "Metagenomic Next-Generation Sequencing of Nasopharyngeal Specimens Collected from Confirmed and Suspect COVID-19 Patients.", MBIO, 20 November 2020 (2020-11-20), XP055968205

## Description

### FIELD OF THE INVENTION

The present inventors have found that susceptibility to SARS-CoV-2 infection is associated with differences in the overall nasal and nasopharyngeal bacterial community structure. The invention utilizes among others the finding that *Corynebacterium spp.* have a major role in the prevention of the infection and that the bacteria decrease the infection rate via multiple mechanisms.

The invention is useful in the prevention of coronavirus infections in the upper respiratory tract.

### BACKGROUND ART

The epithelial cells of the upper and lower respiratory tracts are the primary targets for airborn infections. These cells are covered by complex bacterial communities mainly in the upper respiratory tract that may directly or indirectly interact with coronavirues. The idea that commensal bacteria may also prevent infection by regulating innate and adaptive host immune responses has already been raised in the art. Furthermore, nasopharyngeal or oronasopharyngeal preparations comprising bacteria to alter the composition of the microbiom in the upper respiratory tract have been proposed.

Lappan and Peacock (Lappan & Peacock, 2019. Corynebacterium and Dolosigranulum: future probiotic candidates for upper respiratory tract infections. Microbiology Australia, 40(4), 172-177.) review studies observing the presence of *Corynebacterium* and *Dolosigranulum* in the microbiom of the upper respiratory tract and which suggest their association with a healthy state. The authors advise that commensal *Corynebacterium pseudodiphtheriticum* and *Dolosigranulum pigrum* strains, which are held to be pathogens of the upper respiratory tract, may have protective role in the nasopharyngeal health of children.

Man et al. in their paper published in 2017 also came to the conclusion that *Dolosigranulum* spp. and *Corynebacterium* spp. are potential keynote players in the upper respiratory tract microbiota as "they have been strongly associated with respiratory health and the exclusion of potential pathogens, most notably *Streptococcus pneumoniae*, in several epidemiological and mechanistic studies." (Man et al., 2017. The microbiota of the respiratory tract: Gatekeeper to respiratory health. Nature Reviews Microbiology, 15(5), 259-270.)

The question of the interrelations of the healthy state of the microbiom and serious infections by RNA viruses, among other coronavirus infections have also been raised recently in several studies.

The highly transmissible and pathogenic coronavirus that emerged in late 2019, i.e. severe acute respiratory syndrome coronavirus 2 (SARS-CoV-2) resulting in coronavirus disease 2019 (COVID-19) has caused a pandemic. The importance of this research is more than supported by the fact that by May 11, 2021, a total of 157.362.408 diagnosed cases and 3.277.834 deaths due to COVID-19 have been confirmed by WHO (https://www.who.int/publications/m/item/weekly-epidemiological-update-on-covid-19---11-may-2021).

SARS-CoV-2 infection seems to have a dual nature: tragically lethal in some persons, surprisingly mild in others, and there were even people who were not susceptible to the virus at all.

The epithelial cells of the upper and lower respiratory tracts are the primary targets for SARS-CoV-2 virus infection and replication. Assuming that the intrusion gate is guarded by a commensal microbiome of the nasopharynx, several authors have tested the bacterial components in infected and non-susceptible individuals.

Nardelli et al. (Nardelli et al., 2021. Nasopharyngeal Microbiome Signature in COVID-19 Positive Patients: Can We Definitively Get a Role to Fusobacterium periodonticum? Frontiers in Cellular and Infection Microbiology, 11 (February), 1-7.) and Maio et al. (Maio et al., 2020. Nasopharyngeal Microbiota Profiling of SARS-CoV-2 Infected Patients. 7, 20-23.) found no difference in nasopharyngeal microbiome composition in the samples from COVID-19 patients and virus-negative controls. Rather, it can be concluded from their data that SARS-CoV-2 infection did not significantly alter the composition of the microbiome compared to the microbiome of the average population. Testing larger number of patients and using the 16S rRNA method, Rosas-Salazar et al. (Rosas-Salazar et al., 2021. SARS-CoV-2 infection and viral load are associated with the upper respiratory tract microbiome. J Allergy Clin Immunol. 2021 Apr;147(4):1226-1233.e2*)* reported a mixed picture of Corynebacteria: while some strains were more abundant in SARS-CoV-2 infected subjects, others were more abundant in those not infected with the virus.

Metagenomic next-generation sequencing in contrast to targeted methods also provides valuable information on the composition of the microbiome at the species level. The metagenomic next-generation study of Mostafa et al. (Mostafa et al., 2020. Metagenomic next-generation sequencing of nasopharyngeal specimens collected from confirmed and suspect covid-19 patients. MBio, 11(6), 1-13.) described a statistically significant decrease of incidence of a commensal organism, *Corynebacterium accolens* in the samples of COVID-19-positive patients. The authors mention that there is evidence in the prior art that *C accolens* has a negative association with colonization by Streptococcus pneumonia; emphasize, however, that further studies would be required to conclude the role of these associations in patients with COVID-19 and do not raise that there may be a negative association between the presence of *C. accolens* and SARS-CoV-2 infection. Actually, the decrease of the level of *C. accolens* may well be a result of the infection.

Engen et al. (Nasopharyngeal Microbiota in SARS-CoV-2 Positive and Negative Patients. Biol Proced Online. 2021 Jun 1;23:10.) found a markedly decreased abundance of genera Corynebacterium in COVID-19-positive relative to negative patients.

Rosas-Salazar et al. SARS-CoV-2 infection and viral load are associated with the upper respiratory tract microbiome. J Allergy Clin Immunol. 2021 Apr;147(4):1226-1233.e2. doi: 10.1016/j.jaci.2021.02.001) reported that Corynebacterium amplicon sequence variants were more abundant in individuals without SARS-CoV-2 infection and in those with low viral load during COVID-19 than in individuals with SARS-CoV-2 infection.

Mostafa et al. (Metagenomic Next-Generation Sequencing of Nasopharyngeal Specimens Collected from Confirmed and Suspect COVID-19 Patients. ASM Journals mBio Vol. 11, No. 6) found a higher relative abundance of Propionibacteriaceae and lower Corynebacterium accolens in COVID-19-positive relative to negative individuals.

### BRIEF DESCRIPTION OF THE INVENTION

The invention is defined by the claims.

### ABBREVIATIONS

- ATCC: American Type Culture Collection
- ACE2: Angiotensin I Converting Enzyme 2
- CTAB: Cetyl trimethylammonium bromide
- ELISA: Enzyme Linked Immunosorbent Assay
- MALDI-TOF: Matrix-assisted laser desorption/ionization - Time-of-flight
- NCAIM: National Collection of Agricultural and Industrial Microorganisms
- NGS: Next Generation Sequencing
- SARS-CoV2: Severe acute respiratory syndrome coronavirus 2
- RBD: Receptor binding domain
- rRNA: ribosomal RNA
- HRP: Horse radish peroxidase
- PCR: Polymerase chain reaction
- WGS: Whole Genome Sequencing
- EMEM: Eagle's minimal essential medium
- TMPRSS2: Transmembrane protease, serine 2

### DEFINITIONS

As used herein bacteria are "viable" if, under conditions which comprise an appropriate medium and nutrients and appropriate temperature they are capable of propagation ("growth", if the number of the viable bacteria increases).

A "culture", as used herein, refers to the cultivation of bacteria in an artificial environment, i.e. *in vitro.* Cultivation and thus culturing thus may include maintaining the bacteria in a viable form and/or propagation of the bacteria. Thus "culture" includes bacteria in a medium in which the bacteria are maintained in a viable form, including dried (lyophilized) forms, cultures on solid media and liquid forms as well. The term culture also encompasses the meaning of the term "consortium".

"Sequence identity" (or "homology") is a ratio that describes how similar a query sequence is to a target sequence, i.e. once the two sequences are aligned, how many characters corresponding to residues in the two sequences are identical. Preferably, it is the ratio of characters which match exactly between two different sequences. As the length of the two sequences may not be identical it is important to define the reference sequence and/or segment with a defined length. Preferably, gaps are not counted and, preferably, the measurement is relational to the shorter of the two sequences. The higher the percent identity is, the more significant the match.

Sequence identity may be determined using any known on-line homology algorithm, like the "BLAST" program, publicly available e.g. at the NCBI, USA, (at http://www.ncbi.nlm.nih.gov/BLAST/) or at the EMBL-EBI, EU (https://www.ebi.ac.uk/Tools/sss/ncbiblast/).

"Genetic modification" includes introduction of exogenous and/or endogenous DNA sequences into the genome of a strain, for example, by insertion into the genome of the Corynebacterium strain by vectors, including plasmid DNA, or bacteriophages, as well as genetic manipulation technologies.

The term "formulation" relates to a composition of matter comprising at least one biologically, preferably medically active ingredient, the "active agent" and at least one other substance e.g. a medium and/or excipient or both suitable for administration into a subject, e.g. a mammal or bird. Preferred formulations are formulations for use in the upper respiratory tract of said subject, preferably a homeothermic subject. The term "formulation" and the term "composition" may be used interchangeably in the context of the invention.

The term "administration" as used herein shall include routes of introducing or applying formulation of the invention, to a subject in need thereof to perform their intended function. In particular administration as used herein relates to administration into the upper respiratory tract of a subject, e.g. a mammal or a bird.

The term "subject" as used herein shall refer to a homeothermic (mammalian or avian, preferably mammalian) subject particularly a human being. In particular, the medical use applies to a subject in need of prophylaxis or treatment of a disorder or disease condition in also touching the upper respiratory tract.

The term "patient" includes a subject under medical care, e.g. one that receives either prophylactic or therapeutic treatment.

"Prophylactic treatment" or "prophylaxis" or "prevention", as used herein includes measure or measures on or in respect of a patient to prevent infection of the patient including reduction of infection rate or severity of infection (number of infected cells). The patient may be a non-infected patient or a patient who has been infected but further infection or reinfection is prevented by said measure or measures. Preferably prophylaxis or prevention includes at least one or more or regular administration to a target site, i.e. the upper respiratory tract, in particular the epithelium thereof.

"Treating", "to treat", "treatment" as used herein refer to improving, alleviating, reducing, mitigating a symptom of a disease or condition, e.g. a symptom of an infection. "Treating", "to treat", "treatment" also include reducing the number of viruses which may enter the cells of the patient.

The "upper respiratory tract" refers to the parts of the respiratory system lying above the sternal angle (outside of the thorax) above the vocal folds, or above the cricoid cartilage. Preferably the larynx is sometimes included in the upper respiratory tract. The upper respiratory tract comprises the nasal cavity and paranasal sinuses, the pharynx (including the nasopharynx/epipharynx, oropharynx/mesopharynx and laryngopharynx/hypopharynx) and preferably includes the larynx. In preferred embodiments "upper respiratory tract" refers to the nasal cavity and the nasopharynx.

The term "predetermined time period" means herein the maximum time period during which the formulation of the invention, calculated from providing it in a form in which it is ready for administration, it can be actually administered while still maintaining its quality, i.e. an acceptable effect, i.e. an appropriate portion of the bacteria remain viable and effective, preferably an at least 50%, 60%, or preferably 70%, or more preferably at least 80% in particular at least 90% of the bacteria remains viable.

The expression "infection" is intended to mean an undesired propagation of virus in the cells of a subject.

The term "comprise(s)" or "comprising" or "including" are to be construed herein as having a non-exhaustive meaning and to allow the addition or involvement of further features or method steps or components to anything which comprises the listed features or method steps or components. Such terms can be limited to "consisting essentially of" or "comprising substantially" which is to be understood as consisting of mandatory features or method steps or components listed in a list, e.g. in a claim, whereas allowing to contain additionally other features or method steps or components which do not materially affect the essential characteristics of the use, method, composition or other subject matter.

As used in this specification and the appended claims, the singular forms "a", "an" and "the" include plural references, and should be construed as including the meaning "one or more", unless the content clearly dictates otherwise. In general, it is also to be understood that the terminology used herein is for the purpose of describing particular embodiments only, and is not intended to be limiting.

### BRIEF DESCRIPTION OF THE FIGURES

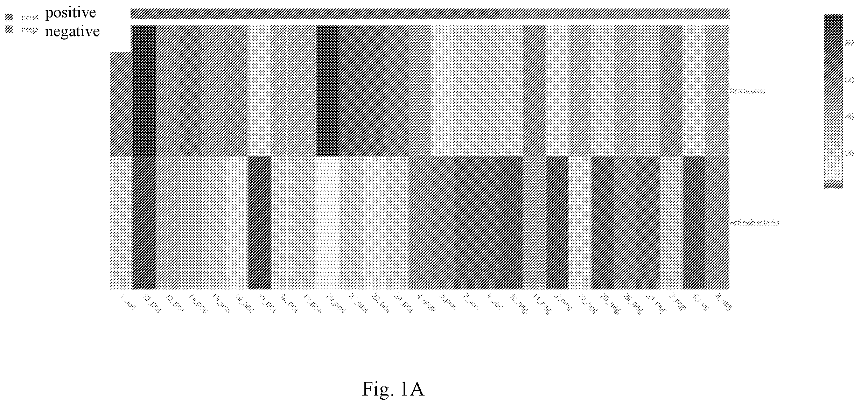

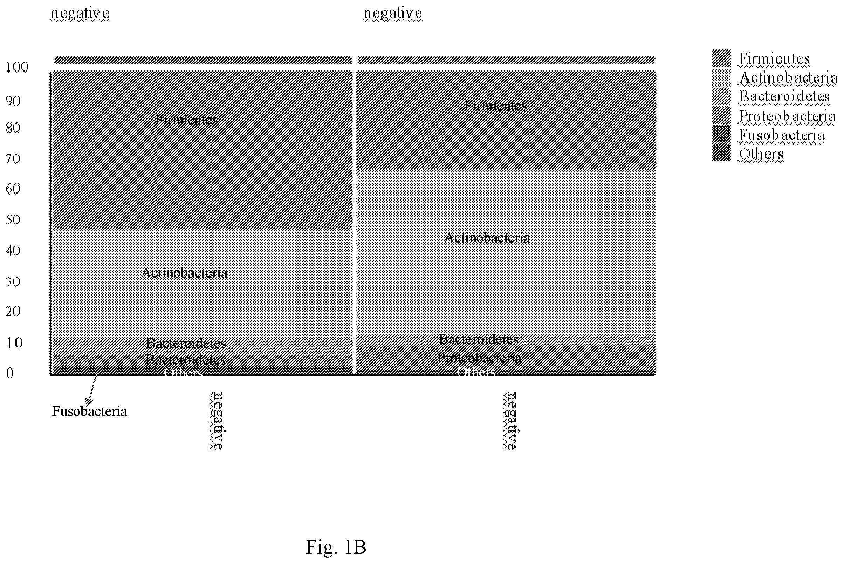

**Figure 1****. Compositional differences in nasopharyngeal microbiota between patients with COVID-19 ("positive") and non-COVID-19 ("negative") subjects.** **Figure 1A** shows the levels of *Firmicutes* and *Actinobacteria* in individual patient samples wherein COVID-19 positive and negative patient samples are indicated. **Figure 1B** shows average relative abundances of microbial phyla detected in nasopharyngeal swabs in COVID-19 positive and negative samples. The datasets generated during the current study are deposited in the NCBI Short Read Archive (SRA) (https://www.ncbi.nlm.nih.gov/sra) under accession number: PRJNA 728384.
**Figure 2****. Compositional differences in nasopharyngeal microbiota between patients with COVID-19 ("positive") and non-COVID-19 ("negative") subjects.** The figure shows average relative abundances of microbial orders detected in nasopharyngeal swabs of positive and negative patient samples.
**Figure 3****. Compositional differences in nasopharyngeal microbiota between patients with COVID-19 and non-COVID-19 subjects.** Average relative abundances of the listed microbial genera detected in nasopharyngeal swabs.
**Figure 4****. Individual differences in genus level in nasopharyngeal microbiota between patients with COVID-19 and non-COVID-19 subjects.** The figure shows the levels of each genera in individual patient samples wherein COVID-19 positive and negative patient samples are indicated.
**Figure 5****. Phylogenetic tree of the Corynebacterium strains isolated from the second study group members' nasopharyngeal samples.** *Corynebacterium sp* KPL 1855 was used as *C. accolens* reference strain (NCBI BioProject:PRJNA224116, NCBI-Taxonomy:1203562)
**Figure 6****. Phylogenetic tree of the *lipS1* of *Corynebacterium* strains isolated from the second study group members' nasopharyngeal samples.** NZ_KI515758.1_281179-282573 0.00466 the *lipS1* of *Corynebacterium sp* KPL 1855 was used as reference (NCBI BioProject:PRJNA224116, NCBI-Taxonomy:1203562)
**Figure 7** **The average ACE2 protein amount of the 24h Caco-2 cell culture and Caco-2 cell-Corynebacterium strain co-cultures**
**Figure 8****. ACE2 mRNA relative expression in Caco-2 cells after infection and co-culture with the *C. accolens* and *C. propinquiun* group - average** Caco-2 cells were infected with *Corynebacterium accolens, Corynebacterium propinquum* for 24 h. Uninfected Caco-2 cells served as negative controls, respectively. The ACE2 mRNA levels were normalized to GAPDH, using quantitative real-time PCR. Results are expressed as mean ± SD of the data from two separate experiments
**Figure 9****. ACE2 mRNA relative expression in Caco-2 cells after infection and co-culture with the selected Corynebacterium spp in each patient sample.** See Figure 8.
**Figure 10****. TMPRSS2 mRNA relative expression in Caco-2 cells after infection with selected Corynebacterium spp. - average** Caco-2 cells were infected with *Corynebacterium accolens* and *Corynebacterium propinquum* strains for 24 h. Uninfected Caco-2 cells served as negative controls, respectively. The TMPRSS2 mRNA levels were normalized to GAPDH, using quantitative real-time PCR. Results are expressed as mean ± error bar of the data from two separate experiments.
**Figure 11****. TMPRSS2 mRNA relative expression in Caco-2 cells after infection with selected Corynebacterium spp.** See Figure 10.

### DETAILED DESCRIPTION OF THE INVENTION

The present inventors have tested the bacterial components in individuals infected by SARS-CoV-2 and a control group of individuals who proved to be non-susceptible in the same setting and found differences in the microbiota composition. After phenomenological description they went after the effect of the strains composing the microbiota and teach that the nasal cavity/pharynx microbiome is a previously unrecognized factor associated with susceptibility to SARS-CoV-2 virus.

Bacterial, specifically *Corynebacterium* cultures and formulations for use against infections of pathogenic viruses, in particular coronaviruses, in the upper respiratory tract of mammals and birds are described.

Coronaviruses are enveloped viruses with a positive-sense single-stranded RNA genome and a nucleocapsid of helical symmetry. They may cause diseases, among others respiratory tract infections in mammals and birds. Coronaviruses belong to the subfamily Orthocoronavirinae, in the family Coronaviridae, order Nidovirales, and realm Riboviria.

There are currently seven coronaviruses known to infect humans, as listed at the European Centre for Disease Prevention and Control (https://www.ecdc.europa.eu/en/covid-19/latest-evidence/coronaviruses): among Alphacoronaviruses: HCoV-OC43 (Sub-genus: Duvinacoronavirus), HCoV-HKU1 (Sub-genus: Setracovirus) and Betacoronaviruses: HCoV-229E (Sub-genus: Embecovirus) cause common colds or lower respiratory tract infections, or in case of HCoV-NL63 ("Betacoronavirus 1"; Sub-genus: Embecovirus), (pseudo) croup and bronchiolitis in children. Further Betacoronaviruses cause more severe respiratory diseases: MERS-CoV the Middle East Respiratory Syndrome (MERS) (Sub-genus: Merbecovirus) and SARS-CoV (or SARS-CoV-1) and SARS-CoV-2 responsible for the Severe Acute Respiratory Syndrome (SARS) (Sub-genus: Serbecovirus). These and other groups of coronaviruses are contemplated as potential targets of the formulations.

Coronaviruses have a small envelope glycoprotein, M, which interacts with the nucleocapsid in the virion, a small E glycoprotein essential for virus budding and a nucleocapsid protein, N, which encapsidates the linear, single-stranded genomic RNA. Cellular entry of enveloped coronaviruses occurs via their viral fusion protein, which is usually the spike (S) protein.

The spike protein binds to specific receptors (entry receptors) on the cell surface, which facilitate viral attachment to the surface of target cells. Entry further requires priming the spike protein by a cellular protease, i.e. cleavage of the S protein, which allows the fusion of the cellular and the viral membranes.

Priming of the S protein comprises multiple steps of proteolytic cleavage of the S protein. Typically the first cleavage of the priming is carried out at the furin cleavage site by a furin and separates an N-terminal and a C-terminal S2 subunits, whereas the N-terminal subunit contains the receptor-binding domain (RBD) responsible for ACE2 binding. Typically the next proteolytic cleavage is carried out by a Type II transmembrane serine protease to activate the S-protein for fusion with an irreversible conformational rearrangement [Fuentes Prior et al. JBC Reviews Priming of SARS-CoV-2 S protein by several membrane-bound serine proteinases could explain enhanced viral infectivity and systemic COVID-19 infection. J. Biol. Chem. (2021) 296 100135].

The main entry receptor for the SARS-CoV and SARS-CoV-2 is angiotensin-converting enzyme 2 (ACE2), an ectopeptidase. The S protein of these viruses are activated by proteolytic cleavage via transmembrane protease/ serine subfamily member 2 (TMPRSS2), a known human airway and alveolar protease [Shulla, Ana, "A Transmembrane Serine Protease Is Linked to the Severe Acute Respiratory Syndrome Coronavirus Receptor and Activates Virus Entry" JOURNAL OF VIROLOGY, Jan. 2011, p. 873-882 Vol. 85, No. 2].

In a particular embodiment the transmembrane protease, preferably TMPRSS2, also activates the receptor protein, preferably ACE2.

It was shown by the present inventors in two different studies that the nasopharyngeal microbiome influences the susceptibility to COVID-19. The first study found that in a closed community of subjects, individuals with significantly higher rates of members of the genus *Corynebacterium* (according to the result of the 16S rRNA sequencing method) in their nasopharyngeal microbiome, were not infected at a local epidemic of SARS-CoV-2.

In the second study longitudinal household transmission of SARS-CoV-2 was examined in order to characterize the potential influence of bacteria on the development of SARS-CoV2 infection. Upon cultivation of nasopharyngeal samples from COVID-infected subjects and uninfected relatives living in a close relationship - close contacts -, a significant difference between the composition of cultured bacteria was found. Surprisingly, *Corynebacterium accolens* was not cultured from any SARS-CoV-2 patients; however, in close contacts who proved to be insusceptible to the virus this species often appeared in pure culture. In contrast, from the samples of COVID-positive patients *Streptococcus mitis* and *Streptococcus sanguis* were often cultured, which never occurred in samples from non-susceptible subjects. To the best of the knowledge of the present inventors, previous studies used members of the average population as negative controls and this is the only study including individuals who were definitely exposed to the virus infection, as negative controls. This situation provided an option to discover the effect of certain bacteria on elements of viral infection.

Based on measurements disclosed herein *Corynebacterium accolens* and *C. propinquum* strains to varying degrees but all significantly reduced the expression of ACE2 and TMPRSS2, both of which are essential for adherence of the virus to cells (Hoffmann et al., 2020. SARS-CoV-2 Cell Entry Depends on ACE2 and TMPRSS2 and Is Blocked by a Clinically Proven Protease Inhibitor. Cell, 181(2), 271-280.e8.).

Based on their measurements but being not bound by this hypothesis the present inventors propose that compounds produced by some *Corynebacterium* strains inhibit, while *Streptococcus* products increase the strength of binding between the SARS-Cov-2 spike protein and the ACE2 receptor.

Lipases may have virucidal activity in case of enveloped viruses, however, this is not necessarily the case (Yu et al., 2020. Broad-spectrum virucidal activity of bacterial secreted lipases against flaviviruses, SARS-CoV-2 and other enveloped viruses. BioRxiv, 2020.05.22.109900.). Bomar et al. (Bomar et al., 2016) have demonstrated that a *C. accolens* strain has antistreptococcal activity through the production of TAG lipase. Using whole genome sequencing of *Corynebacterium* strains grown from COVID negative contacts, the present inventors have also shown that 8 of the 10 *C. accolens* strains found were capable of producing a TAG (Triacylglycerol) lipase enzyme. However, no other *Corynebacterium* species harboured this lipase LipS1 enzyme. *C. accolens* TAG lipase LipS1, which is likely to be extracellular (Bomar et al., 2016. Corynebacterium accolens releases antipneumococcal free fatty acids from human nostril and skin surface triacylglycerols. MBio, 7(1), 1-13.), hydrolyses TAG in the envelope of the *Coronavirus.* This feature definitely provides an advantage to the host against the virus and correlate well with the finding of the greater relative abundance of nasal *Corynebacterium spp.* in individuals who did not get the infection.

*Corynebacterium* and *Streptococcus* strains are often involved in the formation of the normal throat flora (Treerat et al., 2020. Synergism between Corynebacterium and Streptococcus sanguinis reveals new interactions between oral commensals. ISME Journal, 14(5), 1154-1169). The present results show that the presence of nasopharyngeal *Streptococcus* strains has a detrimental effect on *Coronavirus* resistance. Certain *Corynebacterium* strains that are not only able to live in the presence of *Streptococcus* but are also able to gain a growth advantage over them, are more successful in protecting against *Coronavirus.* However, although 8 of the *C. accolens* strains isolated produced the lipase enzyme, only a few were able to inhibit the growth of Streptococcus.

Corynebacterium strains have been deposited in accordance with the Budapest Treaty at the National Collection of Agricultural and Industrial Microorganisms, Institute of Food Science and Technology, Hungarian University of Agriculture and Life Sciences, H-1118 BUDAPEST, Somlói út 14-16, Hungary.

**Table 1. Deposited strains with their date of deposition and accession numbers are as follows.**

| Species | Name given by NCAIM | Name used herein | Accession no. | Date of deposition | Most important features¹ |
|---|---|---|---|---|---|
| *Corynebacterium accolens* | SU001 | C3 | NCAIM (P) B 001495 | June 7, 2021 | comprising a sequence encoding LipS 1 lipase, inhibiting growth of S. mitis and S. sanguis, reducing ACE2 and TMPRSS2 expression |
| *Corynebacterium propinquum* | SU002 | C12 | NCAIM (P) B 001496 | June 9, 2021 | reducing ACE2 and TMPRSS2 expression, cultivation capability in presence of *S. mitis* and *S. sanguis* |
| *Corynebacterium propinquum* | SU003 | C13 | NCAIM (P) B 001497 | June 9, 2021 | reducing ACE2 and TMPRSS2expression, cultivation capability in presence of *S. mitis* and *S. sanguis* |

| | | | | | |
|---|---|---|---|---|---|
| ¹A more detailed characterization of the strains can be found in the Materials and Methods. | | | | | |

To summarize the results, the present inventors have observed patients between May and June 2020, said patients being treated at the Department of Psychiatry and Psychoterapy at Semmelweis University, wherein out of 27 patients in close contact with each other in the psychiatric ward, 16 patients (11 men and 3 women) became infected during the study period, however, despite the close contact, 11 patients (6 women and 5 men) were not infected with the virus. The average number of PCR tests performed per patient during the 1.5 months of the study period was 9.1. Examination of the nasopharyngeal microbiome by 16S rRNA in 27 patients showed that in phylum level significantly more *Firmicutes* and less *Actinobacteria* were observed in the SARS-CoV-2 positive patients. In SARS-CoV-2 negative close contact patients, more *Corynebacterium spp.* and less *Streptococcus spp.* were observed at genus level.

In the other part of the study, between November and December 2020, family members of confirmed COVID-positive patients were included. COVID-positive individuals (n=10) and their families (n=18) were quarantined together and in close contact with each other. SARS-CoV-2 PCR was performed on nasopharyngeal samples from all subjects, and then nasopharyngeal microbiome was determined by 16S PCR metagenomic analysis. Bacteria from the nasopharyngeal samples were cultured. COVID-19 patients had one or more different *Streptococcus* strains, combined with or without *Corynebacterium propinquum* or other bacterial strains. *Corynebacterium accolens* was not cultured from the nasopharyngeal sample of any COVID-19 patient. Pure culture *Corynebacterium accolens* has grown from the nasopharyngeal samples of COVID-19 negative contacts, or these bacteria have been associated with *C. propinquum* and other bacteria. *Streptococcus* strains were not cultured from the nasopharyngeal sample of any COVID-19 negative contact.

Further, in the comprehensive study of all *Corynebacterium spp,* it was observed that all 22 *Corynebacterium* strains significantly reduced the expression of and thus the number of both ACE2 receptors and TMPRSS2 proteins on the surface of co-cultured human Caco-2 cells, however, there was no significant difference between the 10 members of the *C. accolens* group and 12 members of *C. propinquum* group. Both groups downregulated the ACE2 and TMPRSS2 receptors to the same extent.

Reduction of expression of the ACE2 receptors and TMPRSS2 proteins was observed at protein level and also at mRNA level. Thus, reduction of expression includes reduction of the protein number or level and reduction of mRNA level or both. Reduction of expression of the ACE2 receptors and TMPRSS2 proteins is due to downregulation. In particular reduction is significant if it is statistically significant in comparison with a reference level or a reference experiment of the same conditions including the same strain wherein the only difference is the presence or absence of the *Corynebacterium* strain. Preferably the level of reduction is at least 10%, or at least 20% or, in particular, at least 30%.

Preferably the reduction of expression is measured at the protein level, preferably by ELISA, preferably as described in the Examples. The skilled person will be aware that several alternatives to ELISA exist, like fluorescent methods, western-blot, etc.

Preferably the reduction of expression is measured at the mRNA level, preferably by RT-PCR assay, preferably as described in the Examples. The skilled person will be aware that several alternatives to RT-PCR exist, like microarray-based methods, etc.

In addition, the supernatants of broth cultures of *Corynebacterium* strains significantly inhibited the binding of S1 protein and ACE2, in contrast to *Streptococcus* broth culture supernatants that significantly increased the strength of ACE2 and S1 binding. By examining the effect of *Corynebacteria* and *Streptococcus* on each other's growth, some strains of *Corynebacteria* were observed to be capable of inhibiting the growth of *Streptococcus mitis* and *S. sanguis.* Lipase production of 8 *C. accolens* strains was confirmed by screening the whole genome sequences of all 22 *Corynebacteria.*

Inhibition of binding of the viral fusion protein to the protease, preferably the Type II transmembrane serine protease, in particular the TMPRSS2 protein and to the entry receptor, preferably ACE2 can be measured e.g. by a Spike-ACE2 interaction screening assay kit, preferably by a tagged S protein and bound ACE2 proteins (of vica versa) wherein tag can be an antibody, a fluorescent tag or an enzymatic tag etc. and can be detected by any appropriate method e.g. as disclosed in the Examples. The level of inhibition can be observed as a reduction in the binding, e.g. the reduction of the level of signal of detection of the tag. Preferably the level of inhibiton is at least 10%, or at least 20% or, in particular, at least 30%.

As to methodology, conventional culture examinations of patients' nasopharyngeal samples were performed, and the isolated bacteria were identified by MALDI-TOF analysis and whole genome sequencing. The effect of selected bacteria on ACE2 expression and TMPRSS2 expression on Caco-2 cells was examined by ELISA method and RT-PCR. Furthermore, the presence of the lipase gene in the genome of *Corynebacterium spp.* was determined. The synergistic, antagonistic effect of different bacteria isolated from the nasopharyngeal samples were also determined.

In conclusion, the presence of *Corynebacterium spp.* in the nasopharyngeal flora, in particular *C. accolens* strains, and certain *C. propinquum* strains, preferably in consortium with the *C. accolens* strains, could reduce the individual susceptibility to SARS-CoV-2 infection by several mechanisms. In contrast, *Streptococcus* presence in the nasopharyngeal flora may increase the susceptibility to the infection.

*Corynebacterium spp.* described herein, preferably the *C. accolens* strains and/or the *C. propinquum* strains, or preferably a consortium of one or more *C. accolens* strain and/or one or more *C. propinquum* strains together, can reduce the individual susceptibility to SARS-CoV-2 infections through several mechanisms selected from the group consisting of: through downregulation of SARS-CoV-2 receptors: ACE2 and TMPRSS2, through the inhibition of S1 protein and ACE2 receptor binding and, preferably, through lipase production.

In a preferred embodiment said *Corynebacterium* culture is also capable of inhibiting growth of at least one of *Streptococcus mitis* and *Streptococcus sanguis* in co-culture or *in vivo.* Thus, said *Corynebacterium* culture is capable of reducing the level or amount of said *Streptococcus* species in the upper respiratory tract of the subject or prevent propagation thereof.

All these mechanisms together act by inhibiting the binding of SARS-CoV-2 to the host cell and by acting on the lipid envelope of the SARS-CoV-2 virus. These mechanisms could reduce the individual susceptibility to the infection.

The use of *Corynebacterium strain* as a probiotic in the upper respiratory tract preferably in the nasal cavity and/or the nasopharynx and/or the oro/mesopharynx or in the nose is useful for the prevention of the infection or asymptomatic transmission.

Thus, the *Corynebacterium* cultures described herein are for use in preventing upper respiratory tract infections by a coronavirus in a subject. Said subject is a homeothermic (mammalian or avian, preferably mammalian) subject, highly preferably a human subject.

The strain is NCAIM P (B) 001495.

### Formulations and kits

The invention also relates to formulations comprising the *Corynebacterium* culture. The *Corynebacterium* culture may be in any form as far as the *Corynebacteria* are viable and are capable, upon administration, maintaining their viability in the upper respiratory tract or any part thereof. Preferably the bacteria are capable of colonizing, preferably propagating on the epithelial surface of these body parts. It is not necessary for the bacteria to enter into or remain viable in other body parts e.g. in the gastrointestinal tract.

Preferably the *Corynebacteria* are stored in the formulation in a stabilized, non-propagating but viable form, in particular examples are a cryopreserved or a dried form. Preferably the formulation comprises one or more protectants or stabilizers, e.g. one or more carbohydrates. Highly preferably the culture is spray-dried or lyophilized. Preferably in this embodiment the *Corynebacteria* start propagating after administration.

The formulation should be suitable to provide a high concentration of *Corynebacterium* to the subject. Preferably at least 10⁴, 10⁵ or 10^{6,} highly preferably at least 10⁷ or 10⁸ or 10¹⁰ cells should be administered by a unit dose of the formulation.

Formulations of the invention can be combined with one or more ingredients which support *Corynebacterium* viability or propagation, if desired, growth.

The formulations of the invention may comprise a pharmaceutically acceptable carrier. The carrier is physiologically compatible with the upper respiratory tract epithelium to which it is administered.

A carrier can be prepared for in particular intranasal and/or oral administration and may comprise liquid- or gel-forming materials for formulations into liquid or gel forms. The composition of the carrier can be varied so long as it does not interfere significantly with the therapeutic activity of the bacterial strains.

The formulation can be formulated to be suitable for intranasal and/or oral administration.

In an embodiment the nasal formulation is in the form of a liquid for administration in the nasal cavity of the subject. Preferably the fluid is to be administered as nasal drops or nasal lavage. The fluid should provide an appropriate environment for the Corynebacteria culture to maintain viability, thus, it should maintain an appropriate ionic strength and pH. Thus, the liquid may comprise appropriate salts and buffering agents. The liquid also may comprise nutrients.

In an embodiment the formulations of the invention can also be administered by and formulated for inhalative administration. In an embodiment such a formulation is in the form of a liquid or in the form of a powder, wherein liquid forms are preferred. Thus an inhalative formulation can be eg. in the form of inhalable powders, aerosol mixtures, oral inhalation solutions or suspensions, nasal sprays, nasal mists, for use in inhalers, fluid pulsers, atomizers, vaporizers, nebulizers, respirators, transpirators, air masks or insufflators e.g in mechanized form thereof. Preferred inhalative formulations are nasal spray, inhalation solutions, suspension, and spray drug products a guidance for the preparation thereof can be found e.g. as disclosed in the respective FDA Guidance Document ["Nasal Spray and Inhalation Solution, Suspension, and Spray Drug Products - Guidance for Industry", July 2002, Issued by: Center for Drug Evaluation and Research, Docket Number: FDA-1999-D-0060.].

Typically, nasal spray products comprise the active ingredients, here the Corynebacteria, suspended in solutions or mixtures of excipients (e.g., viscosity modifiers, emulsifiers, buffering agents) in nonpressurized dispensers. The dose can be metered by e.g. a spray pump or could have been premetered during manufacture.

In an embodiment the intranasal formulation is in the form of a viscous liquid, a liquid suspension, a paste, a gel or an ointment and can be administered topically on the epithelial surfaces of the nose. In this embodiment the formulation can include one or more gelling agents that can act as an adhesive agent to adhere the formulation to the nasal cavity, pharynx, in particular the nasopharynx or possibly in the oropharynx or throat. In an embodiment the intranasal formulation is in the form of an ointment or gel.

The formulation also can be stored in the form of a dried form, e.g. spray-dried or freeze-dried form, e.g. lyosphylisate and a solution for use in inhalative administration can be prepared before administration by dissolving or suspending the dried formulation. In this case care shall be taken to convert the formulation into a liquid form before the administration not longer than a time-period during which the formulation remains effective e.g. the *Corynebacteria* remain viable or a sufficient portion thereof remains viable.

In a particular embodiment the formulation is in the form of nasal drops. In nasal drops the *Corynebacteria* comprise bacteria in viable form. In a preferred embodiment the bacteria number is stabilized in the formulation and the bacteria begin to be active, e.g. to propagate or divide only when administered.

In a highly preferred embodiment the formulation is in the form of a nasal spray (e.g. atomizer, vaporizer, nebulizer). The nasal spray comprises the bacteria in viable form. The nasal spray may be formulated as a ready to use composition or may be prepared freshly before administration. E.g. lyophilized bacteria may be reconstituted in an appropriate vehicle.

In an embodiment the formulation is present in a kit comprising a compartment which comprises the *Corynebacteria* and an other compartment comprises a medium suitable for administration. Upon administration the *Corynebacteria* and the medium are mixed in a predetermined dose. Thereby the *Corynebacteria* are transferred from a stabilized state into a propagating state.

In an embodiment the *Corynebacteria* become active and propagating in the upper respiratory tract, preferably in the nasal cavity, pharynx, larynx, and/or throat of the host.

In an embodiment the *Corynebacteria* may be microencapsulated and encapsulated bacteria are released in the upper respiratory tract, preferably in the nasal cavity, pharynx, larynx, and/or throat of the host.

In an embodiment the formulation of the invention is present in a device e.g. an applicator of said formulation which is appropriate for the way of administration the formulation is prepared for.

For example if the formulation is a nasal drop it may be present in a dropper, a dropping bottle or a pipette.

If the formulation is an inhalable formulation it may be present in inhalers, fluid pulsers, atomizers, vaporizers, nebulizers, respirators, transpirators, air masks or insufflators e.g in mechanized form thereof.

Alternatively, the device can be packaged together with the formulation so that they together may form a kit.

If the formulation is an ointment or the like the applicator may be a squeeze tube or the like for application of an ointment or the like to the epithelium of the upper respiratory tract e.g. the nasal cavity.

In an embodiment the formulation comprises fatty acids suitable for the growth of *Corynebacterium.*

In a preferred embodiment the formulation comprises one or more protectants or stabilizers, or carbohydrates (sugars), polymers, proteins, amino acids. Carbohydrates may include, among others, sucrose, xyllite, trehalose etc. Another usual additive is skimmed milk. In a preferred embodiment antioxidants are also added. In a particular embodiment applicable drying techniques include lyophilisation (freeze-drying), vacuum-drying, spray-drying, fluidized bed-drying, air-drying, preferably freeze-drying or spray-drying.

Methods for stabilizing the number of bacteria are known for a person skilled in the art [see e.g. Goderska, Kamila "Different Methods of Probiotics Stabilization" in Probiotics, IntechOpen, Published: October 3rd 2012 Edited by Everlon Rigobelo DOI: 10.5772/50313, ISBN: 978-953-51-0776-7].

### Methods of treatment

The formulations can be applied in a therapeutically effective amount to the upper respiratory tract for the treatment and/or prevention of one or more respiratory infections (e.g. an infection by a coronavirus, preferably a SARS coronavirus, highly preferably SARS-CoV-2). A formulation may be dropped or sprayed into the nose or may be inhaled.

Treatment preferably means inducing a reduction in the amount, type, or intensity or duration (or combination thereof) of one or more symptoms of a respiratory infection (e.g. an infection by a coronavirus, preferably a SARS coronavirus, highly preferably SARS-CoV-2) or optionally including prevention thereof.

The culture for use or the formulation may be administered to a subject already infected by the coronavirus to promote the reduction of the number of viruses entering the cells of the subject and/or to reduce the number of *S. mitis* and/or *S. sanguis* thereby reducing the interaction between the ACE2 receptors of the host cells and the S protein of the coronavirus.

Successful prevention or prophylaxis means that substantially no respiratory infection symptoms occur after exposure of the host to the virus as defined herein that is capable of causing respiratory infections; preferably no propagation of the virus can be observed in the nasal cavity or the nasopharyngeal swab of the patient after a pre-determined time normally sufficient for virus propagation (e.g. preferably at least 2 days, at least 5 days or at least 8 days or at least 2 days, at least 5 days or at least 8 days least 10 days in case of SARS-CoV-2).

Once administered, the *Corynebacterium* strains of the culture can form part of the flora (microbiom) of the upper respiratory tract including the nasal cavity and paranasal sinuses, the pharynx (nasopharynx, oropharynx and laryngopharynx) and preferably including the throat and the larynx, and exhibit beneficial prophylactic and/or therapeutic effects in the respiratory tract.

The subject in need thereof may be a subject who has one or more respiratory infections caused by the pathogenic respiratory virus or may be a subject who is exposed or potentially exposed to such viral infection or endangered by such infection or a subject having an increased susceptibility to the respiratory infection.

In one embodiment prevention means prevention in a population of subjects.

Optionally, prior to the administration of the composition the virus causing the respiratory infection can be detected using any detection method known in the art. Those of skill in the art are aware of methods of detection of said viruses. Optionally, prior to the administration of the composition, one or more respiratory infections can be diagnosed in the subject using any methodology known in the art. Diagnosis is the identification of the cause or likely cause of a respiratory infection.

In a preferred embodiment the method comprises obtaining data regarding a therapeutically effective dosage range for treatment or prevention of the one or more respiratory infection symptoms. This may also comprise obtaining data in a particular type of subject and/or determining the effective dosage range for the particular type of subject. This obtaining data may comprise diagnosing the subject with any condition making him or her susceptible for the viral infection. Obtaining data may also comprise data on the microbiom of the subject, in particular, defining the composition of the microbiom in the upper respiratory tract, in particular a sample of nasopharyngeal swab. If the sample comprises a high level of the *Corynebacteria* species and/or a low level of or a lack of *Streptococcus* species a lower dose of the formulation can be administered or maybe there is no need of treatment. If the sample comprises a low level of or a lack of the *Corynebacteria* species and/or a high level of *Streptococcus* species there is a need for treatment or administration of the formulation or there is a need for a higher dose of the formulation to be administered.

Moreover, other data like age, sex, body mass or any index related thereto, lifestyle can be obtained.

In this way the therapeutically effective dose may be administered.

Optionally the effect of the formulation is monitored in the patient and the composition of the microbiom is measured after administration. This allows a decision on the interruption or continuation of the treatment.

In one embodiment the administered Corynebacterium strains are present in the nasal cavity and/or the nasopharynx and/or oropharynx for about 1 day, about 1 week, about 2 weeks, about 3 weeks, about 1 month, about 3 months or about 12 months after administration of the bacteria. In a particular embodiment monitoring comprises taking and analyzing samples in such time after administration or after the starting of the method for treatment.

The formulations can be administered to the upper respiratory tract, including nasal cavity and paranasal sinuses, the pharynx (nasopharynx, oropharynx and laryngopharynx) and preferably including the throat and/or the larynx of a host or subject such as an animal, including a mammal, for example, a human, a non-human primate, a dog, a cat, a horse, a bovine, a goat, or a rabbit. In preferred embodiments the culture for use according to the invention or the formulation is administered to the nasal cavity and/or to the nasopharynx.

In particular the formulations can be administered to the upper respiratory tract, preferably into the nose, or orally one or more times in a week, preferably one or more times a day (daily). Particularly the formulations can be administered to the upper respiratory tract for between about 1 minute and about 8 hours, preferably between 1 hour and about 6 or 4 hours. Repeated administration is preferred to ensure the continuous presence of *Corynebacteria* in the upper respiratory tract.

### Preparation of the formulations

Bacterial cultures can be prepared e.g. as described in the Examples, specifically in the Materials and Methods. Large scale bacterial cultures can be prepared in, for example, a fermenter. *Corynebacteria* may have different rate of propagation (duplication time) and a faster rate is preferred. The bacteria can be harvested from the fermenter and can be, for example, concentrated. Bacteria can be prepared for use by, for example, dehydration, air drying, lyophilizing, freezing, spray-drying as known in the art. Methods for scale-up are known in the art.

### Further aspects

In an embodiment the formulation is stored in the form of a dried form, e.g. spray-dried or freeze-dried form.

In a preferred embodiment the formulation is dropped or sprayed into the nose. In a preferred embodiment the formulation is smeared into the nose. In a preferred embodiment the formulation is inhaled.

In an embodiment the treatment comprises prevention of the respiratory disease or prevention of the induction of symptoms thereof.

Preferably the subject in need of treatment is a subject who has one or more respiratory infections caused by the pathogenic respiratory virus or preferably a subject who is exposed or potentially exposed to such viral infection or endangered by such infection or a subject having an increased susceptibility to the respiratory infection.

Preferably the subject is diagnosed as a subject in need of the treatment.

In a preferred embodiment before treatment a nasopharyngeal sample of the subject is analysed for the presence of *Corynebacteria* species.

Preferably also further subject data indicating potential susceptibility to infection by said virus or develop symptoms are obtained before treatment.

Optionally the effect of the formulation is monitored in the patient.

Optionally treatment is started if the subject is found to be infected or to be susceptible for or preferably endangered by infection.

### EXAMPLES

### Materials and Methods

### Ethical approval and involvement of patients in the study, consent to participate

Sample collection protocols were approved by the Ethics Committee of Semmelweis University (SE RKEB: 217/2020). The present study was conducted in accordance with ethical standards that promote and ensure respect and integrity for all human subjects and the Declaration of Helsinki. All research was performed in accordance with guideline and regulation of Semmelweis University, and written informed consent was obtained from all patients.

In the periods of May-June 2020 and November-December 2020, in 2 waves of the COVID-19 epidemic in Hungary, nasopharyngeal swab samples were collected from 27 and 28 persons, respectively. Of the subjects included in the study, 26 had symptomatic and PCR-confirmed SARS-CoV-2 infection. For the negative control group (29 persons) the following selection criteria were applied: close contact with the infected subject, multiple PCR-negative results during the study period, no symptoms, no vaccination, no previous evidence of COVID-19 infection verified with negative AdviseDx SARS-CoV-2 IgG II CMIA test (Abbott, USA). The first group of persons - inpatients of the Closed Ward of the Semmelweis University Department of Psychiatry and Psychotherapy - participated in a retrospective study. The second experimental group, 10 COVID-positive persons and their family members (18 persons) quarantined together with them, was designed to be selected for the study from outpatients of the Department of Otorhinolaryngology and Head and Neck Surgery, Semmelweis University, and family members living together in close contact with them.

### Culturing methods:

Nasopharyngeal swab samples of the second study group were inoculated on Columbia blood agar (Biolab, Hungary). Antimicrobial susceptibility disc containing 50 µg Fosfomycin (Oxoid, ThermoFischerScientific Inc, Sweden) was used to select *Corynebacterium* strains from other bacterial participants. All cultured bacteria were identified by the MALDI-TOF method (Bruker Daltonik, Germany).

For the detection of bacterial products that affect the ACE2-Spike binding 24 different *Corynebacterium* strains, one *Streptococcus sanguis* and one *Streptococcus mitis* strain previously isolated from the second study group patients' nasopharyngeal swabs were cultured in EMEM medium (Lonza Biosience) at 37°C in an atmosphere containing 5% CO2.

Human colonic epithelial cell line Caco-2 was previously obtained and used for this study. The Caco-2 cells were cultured in EMEM medium (Lonza Biosience) at 37°C in a humidified atmosphere containing 5% CO2 and the medium was changed every two days. No antibiotics were added to allow the co-cultivation of bacteria. The Caco-2 cells were cultivated alone for four days before the addition of the overnight cultures of *Corynebacteria.* Human Caco-2 cells and bacteria were co-cultured for additional 48 hours.

We examined how each *Corynebacterium* strain and the two *Streptococcus* strains mutually influence each other's reproduction. A pure bacterial culture from each bacterial strain was suspended in saline, its turbidity was standardized at 0,5 McFarland, and it was swabbed uniformly across Columbia blood agar plates forming a basic lawn. Another bacterial strain was also inoculated in duplicate line form on the basic bacterial lawn. All *Corynebacterium* strain lines were grafted onto both *Streptococcus* lawns and, conversely, the two *Streptococcus* strains were grafted onto each *Corynebacterium* lawn. The inhibitory effect of lawns on the growth of the lines was investigated.

### DNA isolation, 16S rRNA gene library preparation and MiSeq sequencing

DNA isolation was performed according to manufacturer's protocol by the ZymoBIOMICS DNA Miniprep Kit (Zymo Research Corp., Irvine, USA). Isolated DNA samples were placed at -80 °C until PCR amplification. Concentration of genomic DNA was measured using a Qubit2.0 Fluorometer with Qubit dsDNA HS Assay Kit (Thermo Fisher Scientific, Waltham, MA, USA). Bacterial DNA was amplified with tagged primers covering the V3-V4 region of bacterial 16S rRNA gene. PCR and DNA purification were performed according to Illumina's protocol. PCR product libraries were assessed using the DNA 1000 Kit with Agilent 2100 Bioanalyzer (Agilent Technologies, Waldbronn, Germany). Equimolar concentrations of libraries were pooled and sequenced on the Illumina MiSeq platform (Illumina, San Diego, CA, USA), using MiSeq Reagent Kit v3 (600 cycles PE).

In order to evaluate the contribution of extraneous DNA from reagents, extraction negative controls and PCR negative controls were included in every run. To ensure reproducibility, each sample was independently extracted and sequenced twice.

Raw sequencing data were retrieved from the Illumina BaseSpace and uploaded to the CosmosID Bioinformatics Platform for evaluation (CosmosID Metagenomics Cloud, app.cosmosid.com, CosmosID Inc., www.cosmosid.com).

### Whole Genome Sequencing (WGS)

From 22 Corynebacterium strains genomic DNA were isolated using the NucleoSpin Microbial DNA Kit (Macherey Nagel, Germany). The Next Generation Sequencing (NGS) libraries in both cases were prepared using the Nextera DNA Flex Library Prep Kit according to the manufacturer's instructions (Illumina, Eindhoven, The Netherlands). The libraries were paired-end sequenced on a MiSeq instrument using 250 bp read length (MiSeq Reagent Kit v2, 500-cycles). The fastq files were used to assemble the *de-novo* draft genome sequence using the SPAdes *de novo* genome assembler (version 3.7.1) and the BioNumerics version 7.6 software (Applied Maths NV, Belgium)

### ACE2-Spike binding inhibition

The 24 h broth cultures of 24 different bacterial strains were centrifuged at 8000g for 3 min and the cell free supernatants were tested with the Cayman Chemical (Cayman Chemical Company, USA) SARS-CoV-2 Spike-ACE2 interaction screening assay kit, according to the manufacturer's instructions. Briefly, the assay uses a recombinant rabbit Fc-tagged SARS-CoV2 Spike S1 RBD binded to the plate precoated with a mouse anti rabbit antibody. 50 µL of supernatant was used to test competition of RBD-ACE2 interaction. The test used recombinant His-tagged ACE2 protein, HRP conjugated anti-His antibody and TMB substrate. Results were read at 450 nm wavelength. All samples were assayed in triplicate. The mean OD of the inhibition control reagent included in the kit was considered to cause 100% inhibitor or 0% binding activity, and the mean OD of sterile broth medium was considered as 100 % of the initial activity.

### Detection of ACE2 protein amount of Caco-2 cells after bacterial co-culture with ELISA method

The Human ACE2 (Angiotensin I Converting Enzyme 2) ELISA Kit (Elabscience Biotechnology Inc, USA) was used to determine the ACE2 receptor amount of Caco-2 cell cultures after 24h co-culture with different *Corynebacterium* strains. Protein isolation was prepared with cell lysis method according to the manufacturer's instructions. Briefly, this sandwich ELISA method uses anti-human-specific- ACE2 antibody for plate pre-coating and detection, the latter is HRP conjugated. All samples were assayed in triplicate. Optical density values were measured at 450 nm. The triplicate readings for each standards and samples were averaged, the average zero standard optical density was subtracted, and the actual concentrations were calculated using the standards.

### Examination of ACE2 and TMPRSS2 expression of Caco-2 cells after bacterial co-culture

The Caco-2 cells were cultivated for four days, an overnight culture of *Corynebacteria* were added to the Caco-2 cell lines and for 48 hours were further co-cultivated. The cells were washed by PBS and 0.25% trypsin and centrifuged. The total RNA was isolated by innuPREP RNA Mini Kit 2.0 (Analytik Jena GmbH, Germany) according to manufacturer's instructions. RNA concentrations were determined using a NanoDrop 1000 spectrophotometer (Thermo Fisher Scientific, USA). 10-100 ng of RNA was used for RT-PCR assay performed using the PrimeScript RT reagent kit (Takara Bio, USA) and amplified the resulting cDNA on a qTOWER 3G (Analytik Jena GmbH, Germany) instrument in the presence of selected primers.

The primers for ACE2 were 5'- GGG ATC AGA GAT CGG AAG AAG AAA-3' forward and 5' -AGG AGG TCT GAA CAT CAT CAG TG-3' reverse. The primers for TMPRSS2 were 5'-AAT CGG TGT GTT CGC CTC TAC-3' forward and 5'- CGT AGT TCT CGT TCC AGT CGT-3' reverse. The primers for GAPDH were 5'- CTA CTG GCG CTG GCA AGG CTG T-3' forward, and 5'- GCC ATG AGG TCC ACC ACC CTG CTG-3' reverse.

Relative mRNA expression was calculated by means of the change in cycle threshold (ΔΔCt) method and normalized to the geometric mean of the housekeeping genes GAPDH. Basal mRNA levels of ACE2 and TMPRSS2 were compared with those of the housekeeping gene GAPDH by calculating the difference between their Ct.

### Data availability

The datasets generated during the current study are deposited in the NCBI Short Read Archive (SRA) (https://www.ncbi.nlm.nih.gov/sra) under accession number: PRJNA 728384.

### Statistical analysis

The significance of the difference between relative abundance of taxon levels was calculated with the Mann-Whitney test. For analyzing the difference between the OD levels in ACE2-Spike binding inhibition and ACE2 protein amount ELISA tests two-tailed students t-test was used. In ACE2 and TMPRSS2 expression studies the difference between mRNA levels measured in the different bacterial co-culture groups was calculated by two-tailed students t-test. Statistical significance between cohorts were implemented using Wilcoxon rank sum testing for microbiome alpha diversity and PERMANOVA analysis for beta diversity.

### Results

### Nasopharyngeal microbiome composition of participants of the first study group

Nasopharyngeal microbiome of 27 persons of the first study group being hospitalized due to psychiatric illness in the Psychiatric Clinic was investigated during a local SARS-CoV-2 outbreak. Of the 27 psychiatric patients, 16 became SARS-CoV-2 positive (13 men, 3 women) and 11 (5 men, 6 women) remained negative.

Sequencing the in total 27 nasopharyngeal samples generated an average of 207.894 total sequence reads per sample.

While the overall nasopharyngeal microbiota composition was distinct between the 16 COVID-19 and 11 non-COVID-19 subjects, there were no significant differences in alpha and beta-diversity.

At the **phylum level,** members of the *Firmicutes* were significantly abundant in patients with COVID-19 compared with non-COVID-19 individuals (mean 52,12% vs 32,50%, p<0.01), whereas *Actinobacteria* were more relatively abundant in non-COVID-19 individuals (54,54% vs 36,20%) the difference was also significant (p<0.01) (Figure 1)

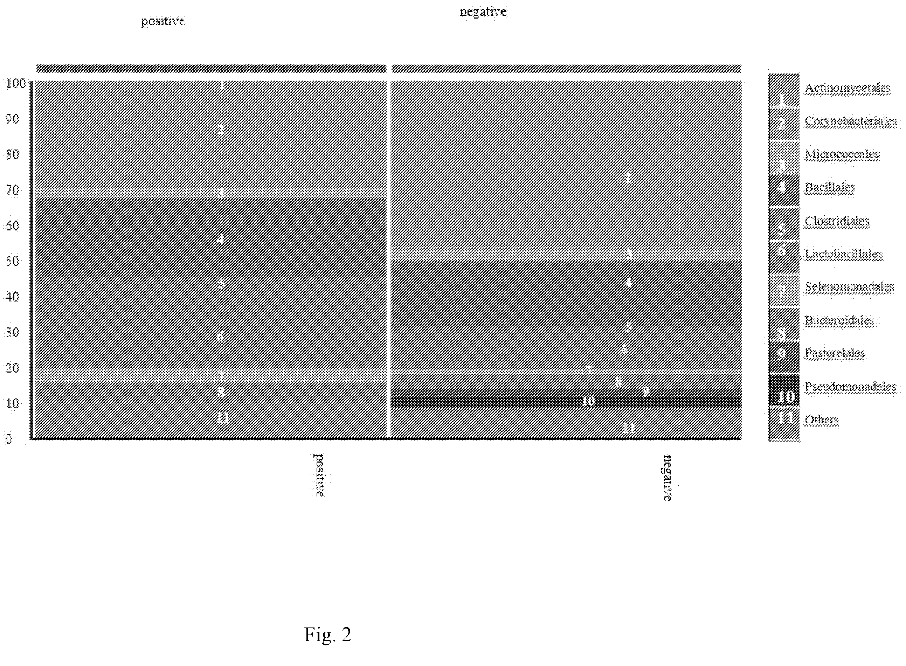

At the order level, members of the *Lactobacillales* order were more relatively abundant in patients with COVID-19 compared with non-COVID-19 individuals (mean 20.00% vs 9.330%), whereas *Corynebacteriales* order were more relatively abundant in non-COVID-19 individuals (46.58% vs 27.92%), however this difference was not significant (Figure 2).

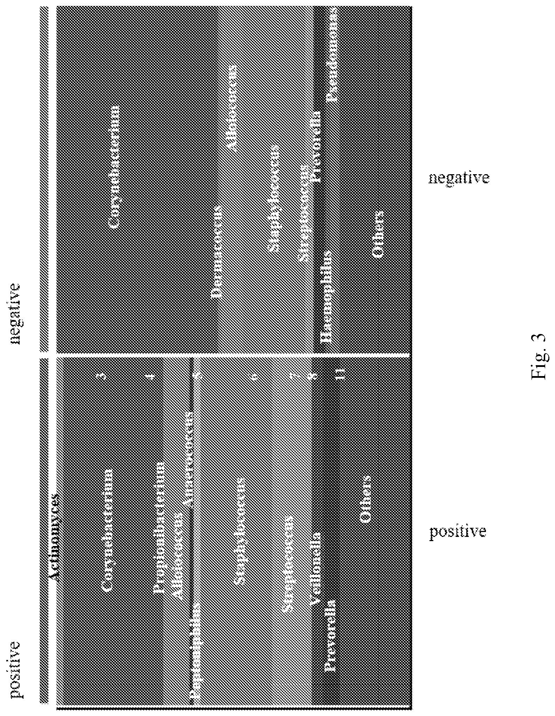

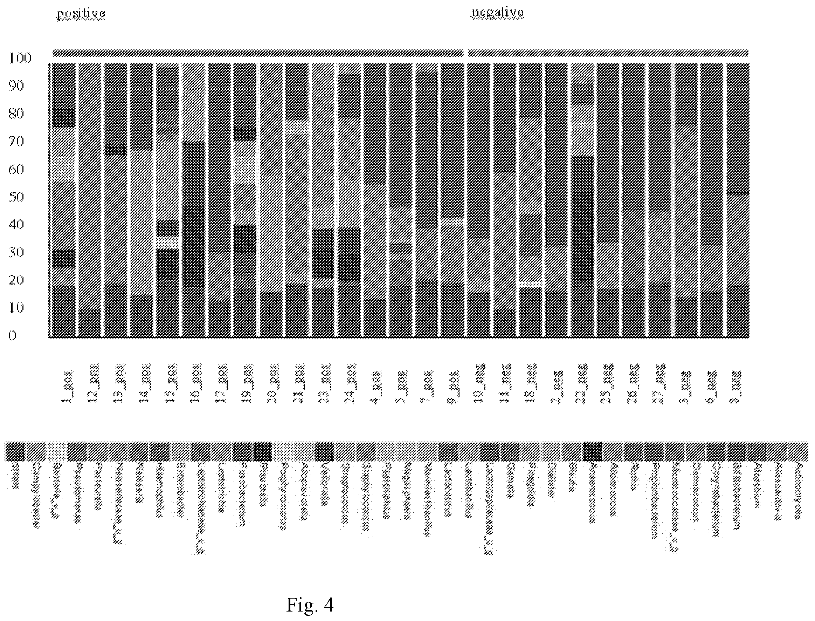

It was a similar observation at genus level that *Corynebacterium* were more abundant in the non-COVID-19 individuals compared to the COVID-19 positive individuals (45.59% vs 27.32%), however, *Streptococcus* were more abundant in COVID-19 positive individuals compared to the non COVID-19 positive individuals (11.2% vs 2.25%,). (Figure 3 and Figure 4)

### Nasopharyngeal swab samples of the second study group patients

Nasopharyngeal swab samples were cultured of 10 COVID-19 patients of the Department of Otorhinolaryngology and Head and Neck Surgery, Semmelweis University, and 18 COVID-19 negative family members living together in closed contact with them.

In total 60 different bacterial strains were isolated from the samples. Table 1 shows the different strains identified with MALDI-TOF. COVID-19 patients had one or more different *Streptococcus* strains, combined with or without *Corynebacterium propinquum* or other bacterial strains. *Corynebacterium accolens* were not cultured from the nasopharyngeal sample of any COVID19 patient. Pure culture *Corynebacterium accolens* has grown from the nasopharyngeal samples of the majority of COVID-19 negative contacts, and in certain cases these bacteria have been associated with *C. propinquum* and other bacteria. *Corynebacterium propinquum* in 2 patients, *S. aureus* in 3 patients, *S. epidermidis* in 2 patients and *Pseudomonas aeruginosa* in 1 patient has grown alone as a pure culture from the COVID-19 negative patients' samples. *Streptococcus* strains were not cultured from the nasopharyngeal sample of any COVID-19 negative patient. Since *S. aureus* and *P. aeruginosa* are considered as facultative pathogens, and no correlation between carriage of *S. epidermidis* and COVID-19 infection was confirmed, henceforth, only *C. accolens, C propinquum* and *Streptococcus* strains were investigated.

### Results of whole genome sequencing (WGS)

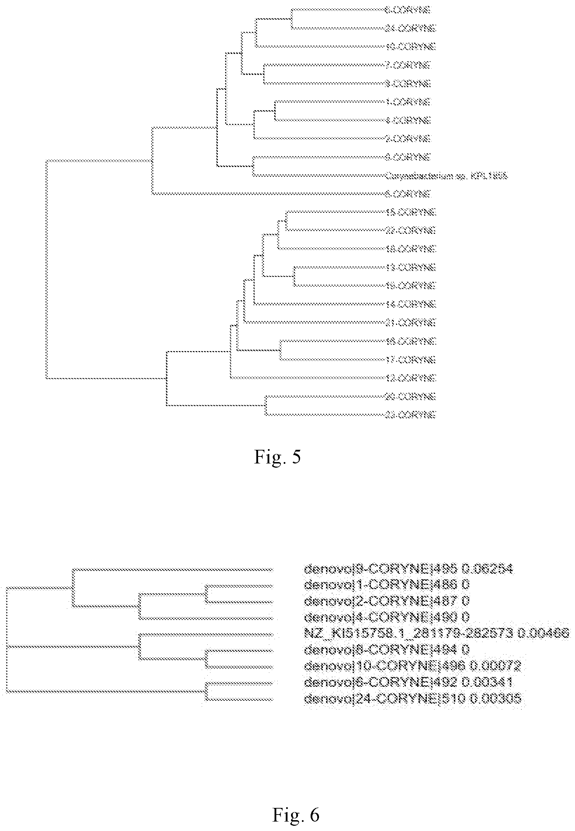

The Whole Genome Sequencing of 22 *Corynebacterium* strains isolated from nasopharyngeal swab samples of COVID-19 negative family members of the second study group resulted in two phylogenetically different groups of bacteria, *C. accolens* group contained 9 *C. accolens* and 1 *C. tuberculostearicum* strain, *C. propinquum* group consisted of 11 *C. propinquum* and 1 *C. pseudodiphteriae* strains. (Figure 5)

The strains have been denominated a C1 to C24 wherein C2 and C3 were identical.

**Table 2 Bacterial strains isolated from nasopharyngeal swab samples of COVID-19 and non-COVID-19 subjects. The numbers indicate the number of patients from the nasopharyngeal samples of whom the given bacteria were cultured.**

| | *C. accolens* group | *C. propinquum* group | S. aureus | S. epidermidis | *Streptococcus mitis or sanguis* | Other |
|---|---|---|---|---|---|---|
| COVID-19 positive patients (10) | 0 | 4 | 0 | 4 | 16 | 3 |
| | | *C. propinquum (4)* | | | | |
| COVID-19 negative patients (18) | 10 | 8 | 10 | 2 | 0 | 3 |
| | *C. accolens (9)* | *C. propinquum* | | | | |
| | *C. tuberculostearicum (1)* | *7* | | | | |
| | | *C. pseudodiphteriae (1)* | | | | |

Based on the Whole Genome Sequencing of 22 *Corynebacterium* strains, the resulted data set also has been screened for the presence of lipase LipS1 enzyme. Eight of ten *C. accolens* harboured this enzyme. More than 99,9% similarity was found among the lipase LipS1 enzyme sequences from the different strains. However, no other *Corynebacterium* species harboured lipase LipS 1 enzyme. (Figure 6)

### ACE2-Spike binding inhibition

Of the 10 *C. accolens* strains tested, the composition of the supernatants from cultures C1, C3, C5, and C10 significantly (p≤0,05) reduced the strength of ACE2-S1 binding, the other 6 strains had no effect on binding activity. Of the 12 *C. propinquum* strains tested, C15, C16, C22, and C23 significantly reduced the strength of ACE2-S1 binding, however *S. sanguis* and *S. mitis* strains significantly increased binding activity (Table2).

**Table 3 ACE2-Spike binding activity of different bacterial strains - Relative binding activities are indicated in comparison with sterile broth medium as 100% (positive control) and binding activity in the presence of inhibition control reagent was considered as 0% binding (negative control). Binding activity in the presence of Streptococcus strains was stronger that with the sterile broth.**

| Sample | Binding activity % | | Mean OD±SD | p value |
|---|---|---|---|---|
| | General | Individual strains | | |
| Sterile broth medium | 100% | | 0.6006±0.0086 | |
| Inhibition control reagent | 0% | | 0.0847±0.0042 | |
| *Corynebacterium accolens* group strains (10 strains) | 89-100% | **C1: 91%** | 0.5542±0.0012 | 0,0136 |
| | | **C3: 89%** | 0.5438±0.0016 | 0,0083 |
| C1 to C10, and C23 | | **C5: 93%** | 0.5644±0.0077 | 0,0104 |
| C2 and C3 being identical | | **C24: 91%** | 0.5541±0.0058 | 0,0039 |
| *Corynebacterium propinquum* group strains (12 strains) C12 to C23 | 79-100% | **C15: 81%** | 0.5026±0.0043 | 0,0006 |
| | | **C16: 90%** | 0.5490±0.0090 | 0,0041 |
| | | **C22: 94%** | 0.5696±0.0086 | 0,0212 |
| | | **C23: 79%** | 0.4922±0.0020 | 0,0017 |
| *Streptococcus sanguis* | 123% | | 0.7192±0,0020 | 0,0014 |
| *Streptococcus mitis* | 138% | | 0.7966±0,00047 | 0,00005 |

### Detection of ACE2 protein amount of Caco-2 cells after bacterial co-culture with ELISA method

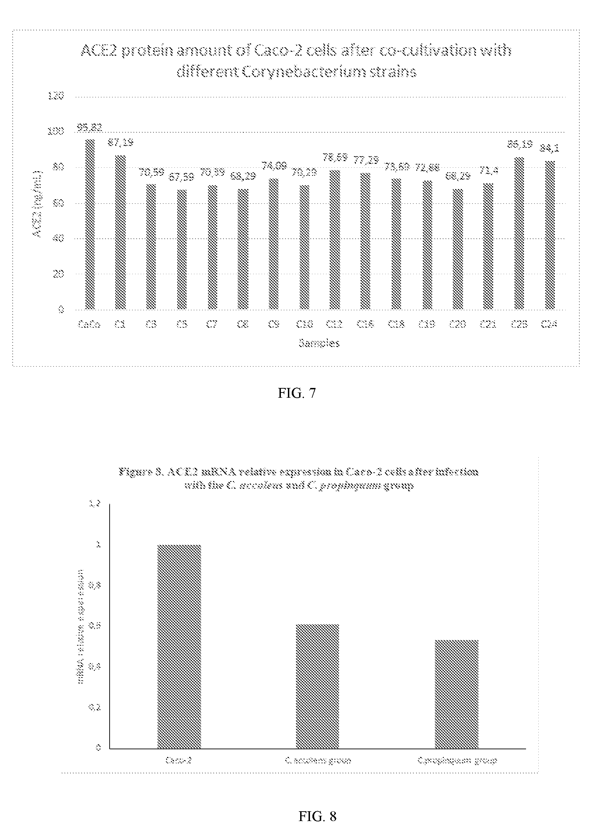

The average ACE2 protein amount of the 24 h Caco-2 cell culture was 95,82 ng/mL. Co-culture with each *Corynebacterium* strain had a decreasing effect on the measurable amount of ACE2. Figure 7 shows that the amount of ACE2 decreased to a level of between 67,59 ng/mL and 87,19 ng/mL after *Corynebacterium* co-culture. Bacterial strains caused varying degrees, but all had a significant inhibitory effect on ACE2 production by Caco-2 cells. The values were near to the lower end of the range of the assay used and therefore the absolute value carries some uncertainty, however, the results clearly show the tendency of each *Corynebacterium* strain to lower the level of ACE2 protein in co-culture.

### The effect of bacteria on the growth of other bacteria

Table 3 shows how the different *Corynebacterium* strains could grow on the *Streptococcus mitis* or *Streptococcus sanguis* lawns. *S. mitis* inhibited the growth of 6 *Corynebacterium strains* (C4, C9, C10, C14, C20, C24), but *S. sanguis* had this inhibiting effect only against 2 of these strains (C9, C24). C3 and C5 *Corynebacterium* strains inhibited the growth of both *S. mitis* and *S sanguis* strains, and C1 *Corynebacterium* only inhibited the growth of *S. mitis.*

**Table 4. Effect of bacteria on each other's growth**

| *Corynebacterium* strain | Growth on *S. mitis* lawn | Growth on *S. sanguis* lawn | Inhibition of the growth of *S. mitis* | Inhibition of the growth of *S. sanguis* |
|---|---|---|---|---|
| C1 | Yes | Yes | **Yes** | No |
| C3 | Yes | Yes | **Yes** | **Yes** |
| C4 | No | Yes | No | No |
| C5 | Yes | Yes | **Yes** | **Yes** |
| C6 | Yes | Yes | No | No |
| C7 | Yes | Yes | No | No |
| C8 | Yes | Yes | No | No |
| C9 | No | No | No | No |
| C10 | No | Yes | No | No |
| C12 | Yes | Yes | No | No |
| C13 | Yes | Yes | No | No |
| C14 | No | Yes | No | No |
| C15 | Yes | Yes | No | No |
| C16 | Yes | Yes | No | No |
| C17 | Yes | Yes | No | No |
| C18 | Yes | Yes | No | No |
| C19 | Yes | Yes | No | No |
| C20 | No | Yes | No | No |
| C21 | Yes | Yes | No | No |
| C22 | Yes | Yes | No | No |
| C23 | Yes | Yes | No | No |
| C24 | No | No | No | No |

### ACE2/ TMPRSS2 expression

### The effect of Corynebacteria on the relative expression of ACE2 receptor on human cells

The relative expression of ACE2 mRNA was significantly decreased in the cells incubated together with *Corynebacterium* spp. compared to uninfected Caco-2 cells after 24 h of the co-culture. (p<0.005).

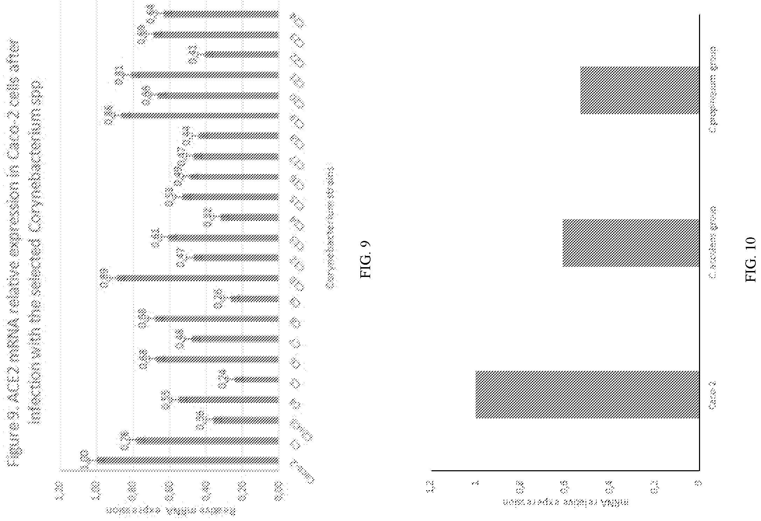

Bacteria belonged *C. accolens* group (n=10) - significantly (p=0,000143) downregulated the ACE2 mRNA expression to the mean 0.61% (±0.22%) compared to Caco-2. The *C. propinquum* group (n=12) also significantly (p=5,08175E-07) downregulated the ACE2 mRNA expression to 0.53% (±0.16%) compared to Caco-2 (**Figure 8**). However, there was no significant difference among the various *Corynebacterium* spp. in the reduction of the ACE2 receptor down-regulation regard (**Figure 9**).

### The effect of Corynebacteria on the relative expression of TMPRSS2 receptor on human cells

The relative expression of TMPRSS2 mRNA was significantly decreased in the cells incubated with *Corynebacterium* spp. compared to uninfected Caco-2 cells after 24 h of the incubation (p= 1,5977E-12).

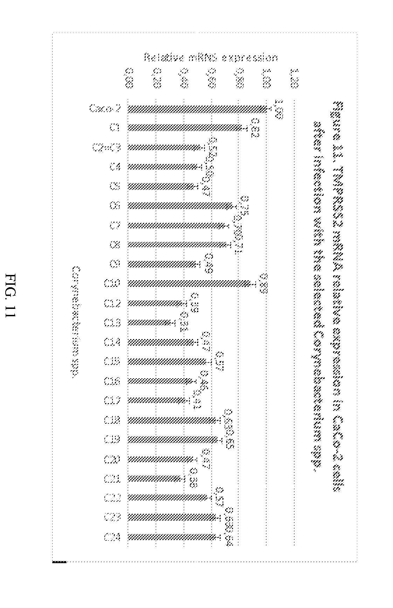

Bacteria which belonged to the *C. accolens* group (n=10) significantly (p= 3,87408E-05) downregulated the TMPRSS2 mRNA expression to the mean value of 0.67% (±0.15%) compared to Caco-2. The *C. propinquum* group species (n=12) also significantly (p= 1,22584E-09) downregulated the TMPRSS2 mRNA expression to mean value of 0.47% (±0.11%) compared to Caco-2. (**Figure 10**). However, there was no significant difference among the various *Corynebacterium* spp. in the reduction of the TMPRSS2 receptor down-regulation regard (**Figure 11**.).

### INDUSTRIAL APPLICABILITY

COVID-19 is, despite the currently available vaccination, is still an ongoing pandemia with high mortality. We still do not have information about the long term efficiency of the different vaccinations, but it is already known that asymptomatic carriage can be occur in vaccinated individuals as well.

The inventors had the option to encounter very special situation - close contact of SARS-CoV-2 positive patients, which provided an opportunity to study patients who were susceptible and who were resistant to SARS-Cov-2 infection under the very same conditions. The present inventors have demonstrated that SARS-CoV-2 susceptibility is associated with differences in the overall nasal bacterial community structure. It was found that *Corynebacterium spp. preferably C accolens* has a major role in the prevention of infection and that the bacteria decrease the infection rate via multiple mechanisms. These mechanisms suggest a causal relationship between the respiratory microbiome and SARS-CoV-2 virus infection.

## Claims

1. Corynebacterium culture or a formulation comprising the Corynebacterium culture for use in the prevention of an infection by SARS-CoV-2, wherein the Corynebacterium culture comprises
*Corynebacterium accolens* SU001 strain having the accession number NCAIM P (B) 001495.

2. A composition comprising at least one Corynebacterium strain, wherein the Corynebacterium strain is *Corynebacterium accolens* SU001 strain having the accession number NCAIM P (B) 001495.

3. The Corynebacterium culture or the formulation for use according to claim 1, wherein the Corynebacterium culture or the formulation is administered to the upper respiratory tract of a subject, preferably to the nasal cavity and/or nasopharynx and/or oro/mesopharynx.

4. The formulation for use according to claim 1 or 3 or the composition according to claim 2, which is a probiotic formulation or a pharmaceutical formulation, optionally comprising an excipient suitable for a probiotic formulation or a pharmaceutically acceptable excipient.

## Patentansprüche

1. Corynebacterium-Kultur oder die Corynebacterium-Kultur enthaltende Formulierung, zur Verwendung bei der Prävention einer Infektion mit SARS-CoV-2, wobei die Corynebacterium-Kultur den Stamm Corynebacterium accolens SU001 mit der Zugangsnummer NCAIM P (B) 001495 umfasst.

2. Zusammensetzung, die mindestens einen Corynebacterium-Stamm enthält, wobei der Corynebacterium-Stamm der Stamm Corynebacterium accolens SU001 mit der Zugangsnummer NCAIM P (B) 001495 ist.

3. Die Corynebacterium-Kultur oder die Formulierung zur Verwendung gemäß Anspruch 1, wobei die Corynebacterium-Kultur oder die Formulierung in die oberen Atemwege, vorzugsweise in die Nasenhöhle und/oder den Nasopharynx und/oder den Oro-/Mesopharynx eines Subjekts verabreicht wird.

4. Die Formulierung zur Verwendung gemäß Anspruch 1 oder 3 oder die Zusammensetzung gemäß Anspruch 2, die eine probiotische Formulierung oder eine pharmazeutische Formulierung ist, gegebenenfalls umfassend einen für eine probiotische Formulierung geeigneten Hilfsstoff oder einen pharmazeutisch verträglichen Hilfsstoff.

## Revendications

1. Culture de Corynebacterium ou formulation comprenant la culture de Corynebacterium, pour une utilisation dans la prévention d'une infection par le SARS-CoV-2, dans laquelle la culture de Corynebacterium comprend
la souche de *Corynebacterium accolens* SU001 ayant le numéro d'accession NCAIM P (B) 001495.

2. Composition comprenant au moins une souche de Corynebacterium, dans laquelle la souche de Corynebacterium est la souche de *Corynebacterium accolens* SU001 ayant le numéro d'accession NCAIM P (B) 001495.

3. Culture de Corynebacterium ou formulation pour une utilisation selon la revendication 1, dans laquelle la culture de Corynebacterium ou la formulation est administrée dans les voies respiratoires supérieures d'un sujet, de préférence dans la cavité nasale et/ou le nasopharynx et/ou l'oro/mésopharynx.

4. Formulation pour une utilisation selon la revendication 1 ou 3 ou composition selon la revendication 2, qui est une formulation probiotique ou une formulation pharmaceutique, comprenant éventuellement un excipient approprié pour une formulation probiotique ou un excipient pharmaceutiquement acceptable.
